# FASCICULE DE BREVET EUROPEEN

(11) **EP 2 139 609 B1**
(45) Date de publication et mention de la délivrance du brevet: **18.09.2013**
(21) Numéro de dépôt: 08787812.0
(22) Date de dépôt: 19.03.2008
(51) Int. Cl.: B05B 17/00

(54) **APPAREILS DE PRODUCTION D'UN GAZ CHARGÉ D'UN COMPOSÉ VOLATIL.**
VORRICHTUNG ZUR ERZEUGUNG VON FLÜSSIGKEITSNEBEL
APPARATUS FOR PRODUCING A GAS CHARGED WITH A VOLATILE COMPOUND

(30) Priorité: 26.03.2007 FR 0702162
(43) Date de publication de la demande: 06.01.2010
(73) Titulaire: Holiste Laboratoires et Développement, 71110 Artaix-Marcigny (FR)
(72) Inventeur: DELANEF, Marie-Laure, F-71110 Artaix (FR); COLLAUDIN, Jean-Michel, F-71800 Saint-Germain-en-Brionnais (FR)
(74) Mandataire: Flavenot, Bernard
(86) Numéro de dépôt international: PCT/FR2008/000358
(87) Numéro de publication internationale: WO 2008/135651

(56) Documents cités:
- DE-A1- 3 049 244
- FR-A- 2 654 273
- GB-A- 512 586

## Description

La présente invention concerne les appareils de production d'un gaz chargé d'un composé volatil comme par exemple, mais non exclusivement, un dérivé organique oxonium, et plus particulièrement du peroxyde de terpène oxonium en vue par exemple de son inhalation.

Il est connu, notamment par le EP-A-0 346 552, un appareil pour la production de peroxydes de terpène oxoniums caractérisé par le fait qu'il est initialement porté à une température relativement élevée, puis refroidi par un courant gazeux chargé de vapeurs de dérivés terpéniques d'oléorésines, jusqu'à une température comprise entre 35°C et 280°C. Le document DE 30 49 244 divulgue un appareil de production de gaz chargé d'un composé en accord avec le préambule de la revendication 1.

Cet appareil comporte un générateur d'arc électrique et/ou un générateur de rayonnement ultra-violet et/ou, très avantageusement, une combinaison de ces deux générateurs. Il est ainsi produit un courant gazeux chargé de vapeurs de dérivés terpéniques d'oléorésines et soumis à l'action d'un arc électrique et/ou d'un rayonnement ultra-violet. Il est aussi possible de soumettre le gaz chargé à l'action d'un générateur de chaleur comme une résistance électrique.

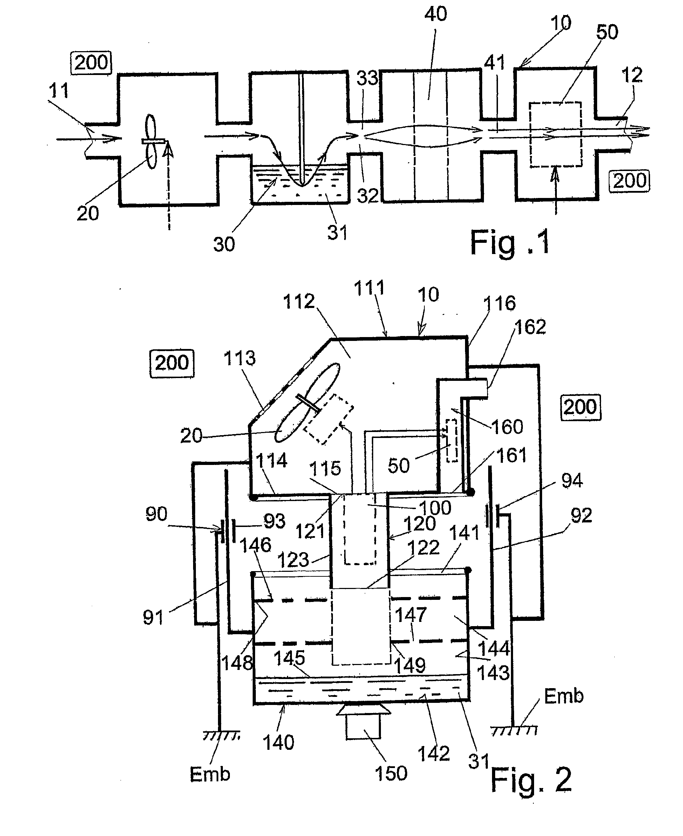

Il est connu un autre appareil comme celui décrit dans le GB-A-512 586, qui comporte une pluralité de bacs ou enveloppes montés en série et positionnés les uns par rapport aux autres en étant répartis sur une hélice ou analogue de façon à remplacer leur encombrement linéaire (voir la figure 5) par un encombrement en enroulement (voir les figures 1 à 4). Or, cet agencement n'a pas pour conséquence une réduction de l'encombrement total, mais simplement sa transformation, et, en outre, ne confère pas à l'appareil une apparence esthétique.

Aussi, la présente invention a-t-elle pour but de réaliser un appareil de production d'un gaz chargé d'un composé volatil, comme par exemple mais non exclusivement un dérivé organique oxonium, et plus particulièrement de peroxyde de terpène oxonium, qui puisse être inhalé, qui pallie au moins en grande partie les inconvénients mentionnés ci-dessus des appareils similaires de l'art antérieur, en réalisant une structure plus compacte et plus esthétique que celle des appareils similaires de l'art antérieur, tout en facilitant son utilisation.

Plus précisément, la présente invention a pour objet un appareil de production, d'un gaz chargé d'un composé volatil, tel que défini dans la revendication 1 annexée.

D'autres caractéristiques et avantages de l'invention apparaîtront au cours de la description suivante donnée en regard des dessins annexés à titre illustratif mais nullement limitatif, dans lesquels :
La figure 1 représente le schéma bloc fonctionnel de l'appareil de production d'un gaz chargé d'un composé volatil selon l'invention,
La figure 2 est une vue schématique d'un mode de réalisation préférentiel de l'appareil selon l'invention, et
Les figures 3 et 4 représentent respectivement deux vues en coupe orthogonales d'un mode de réalisation industriel de l'appareil selon l'invention conforme à la représentation selon la figure 2.

Il est tout d'abord précisé que, sur les figures, les mêmes références désignent les mêmes éléments, quelle que soit la figure sur laquelle elles apparaissent et quelle que soit la forme de représentation de ces éléments.

Par référence plus particulièrement à la figure 1 qui se présente sous la forme d'un bloc diagramme, l'appareil de production d'un gaz chargé d'un composé volatil selon l'invention, par exemple un dérivé organique oxonium et plus particulièrement de peroxyde de terpène oxonium en vue de réaliser notamment des inhalations, comporte un conduit fluidique 10 comprenant une entrée 11 et une sortie 12.

L'appareil comporte en outre, toujours plus particulièrement par référence à la figure 1, montés successivement en série dans le conduit fluidique 10 entre son entrée 11 et sa sortie 12, un aspirateur 20 pour aspirer un gaz porteur disponible à l'entrée 11 du conduit fluidique 10 et le refouler vers la sortie 12 de ce conduit fluidique, un barboteur 30 pour faire passer le gaz porteur dans un liquide volatil 31 contenu dans ce barboteur de façon à obtenir en sortie 32 du barboteur un flux du gaz porteur 33 chargé en vapeurs du liquide, un dispositif de fractionnement 40 du flux gazeux chargé obtenu en sortie 32 du barboteur 30 pour délivrer à sa sortie 41 le flux gazeux chargé sous une forme fractionnée, et un organe de traitement 50 du flux gazeux chargé fractionné pour lui donner une qualité déterminée avant qu'il ne soit éjecté à la sortie 12 du conduit fluidique 10.

Au sens de la présente description, le terme "aspirateur" recouvre la définition de tout dispositif, quels que soient son fonctionnement et sa structure, pour aspirer un gaz en un premier point d'un circuit et le refouler en un second point dans le but d'obtenir une circulation de ce gaz, c'est-à-dire un aspirateur proprement dit mais aussi par exemple une turbine, un ventilateur, un refouleur, etc.

De même, au sens de cette même description, par "barboteur" il doit être compris tout dispositif qui permet à un gaz de passer dans un liquide et, en y passant, de se charger de vapeurs de ce liquide, et ce à quelques température et/ou pression et de quelque façon que ce soit, que ce liquide soit statique et/ou dynamique comme par exemple avec une distribution de ce liquide sous la forme d'un goutte à goutte ou analogue.

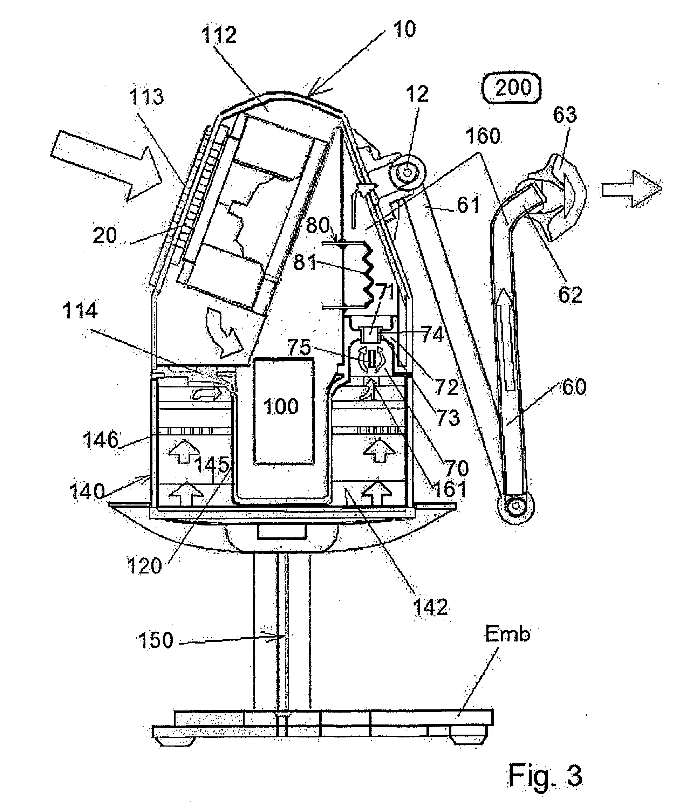

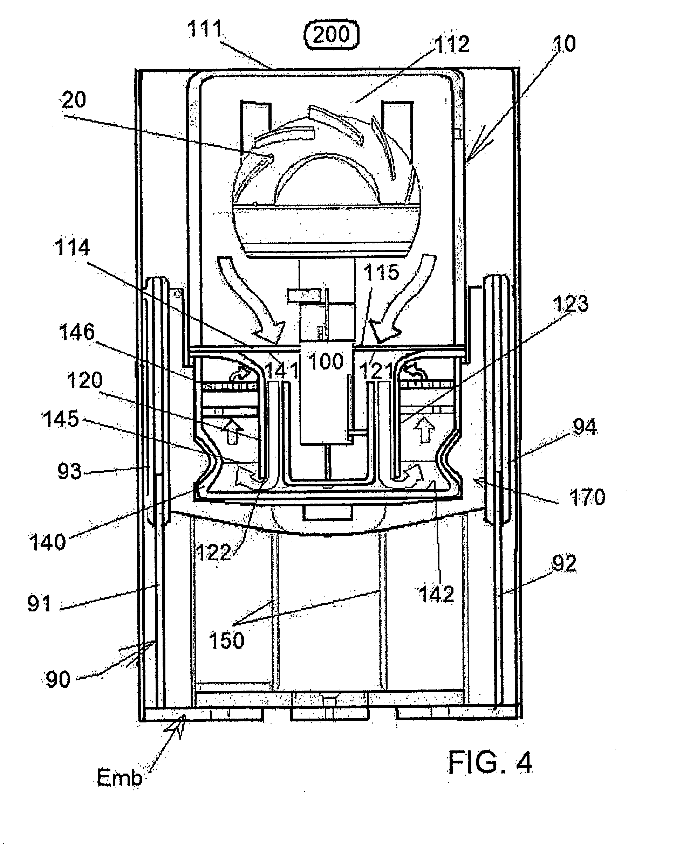

Selon un mode de réalisation préférentiel tel qu'illustré de façon schématique sur la figure 2 et selon le mode de réalisation industriel illustré sur les figures 3 et 4, le conduit fluidique 10 comporte au moins une enceinte 111 apte à être positionnée verticalement, comprenant une première ouverture 113 située sensiblement en sa partie supérieure 112, cette première ouverture constituant l'entrée 11 du conduit fluidique 10, et un fond 114 relativement plan dans lequel est réalisée, sensiblement en son centre, au moins une deuxième ouverture 115.

Le conduit fluidique 10 comporte en outre une tubulure 120 avantageusement verticale comme illustré et d'une hauteur selon la verticale égale à "L", comprenant à ses deux extrémités, respectivement une troisième ouverture 121 située en position haute et une quatrième ouverture 122 située en position basse, cette tubulure 120 étant solidarisée avec le fond 114 de l'enceinte 111 de façon que les deuxième et troisième ouvertures 115, 121 soient en communication de façon étanche.

Le conduit fluidique 10 comporte aussi un flacon 140 comportant en partie haute une cinquième ouverture 141 d'une forme sensiblement complémentaire du fond 114 de l'enceinte 111, ce flacon ayant une profondeur selon la verticale supérieure à la hauteur "L" de la tubulure 120, et un canal de sortie 160 comportant une sixième 161 et une septième 162 ouverture, le canal de sortie étant monté en coopération avec l'enceinte 111 et le flacon 140 de façon que la sixième ouverture 161 soit apte à être en communication étanche avec la cinquième ouverture 141, par exemple au moyen d'un joint annulaire ou analogue comme évoqué sur les figures 2 à 4, et que la septième ouverture 162 débouche dans le milieu ambiant 200, cette septième ouverture 160 constituant la sortie 12 du conduit fluidique 10.

Il est utilisé ci-dessus une notion de verticalité pour l'enceinte et la tubulure. Cependant, bien que définissant une réalisation préférentielle, cette notion de verticalité ne doit pas être prise comme une limitation au positionnement de l'enceinte et de la tubulure, mais comme une facilité d'expression pour définir les différents niveaux, notamment des ouvertures.

De façon préférentielle, comme plus particulièrement visible et schématiquement illustré sur les figures 2 à 4, l'appareil comporte en outre des moyens 150 pour commander le déplacement du flacon 140 entre deux première et seconde positions stables, la première position étant une position dans laquelle la cinquième ouverture 141 du flacon est éloignée du fond 114 de l'enceinte 111, figure 2, et la seconde position étant celle dans laquelle la cinquième ouverture 141 du flacon 140 est sensiblement confondue de façon étanche avec ce fond 114, figures 3 et 4, de façon que la tubulure 120 plonge dans le flacon 140 sans atteindre son fond 142 pour laisser, entre sa quatrième ouverture 122 et le fond 142 du flacon, une distance non nulle dans un but qui sera explicité ci-après, et de façon en outre que, entre la paroi latérale extérieure 123 de la tubulure 120 et la paroi latérale intérieure 143 du flacon 140, soit défini un espace annulaire 144 pour y loger, comme il sera aussi défini ci-après, un élément constitutif de l'appareil selon l'invention

Ces moyens 150 de commande de déplacement du flacon 140 peuvent être de tout type, par exemple manuels avec un levier ou analogue, mais aussi constitués d'un système apte à être commandé électriquement, par exemple un vérin ou analogue.

De façon très avantageuse, quand le conduit fluidique 10 a une structure comme celle définie ci-dessus, l'aspirateur 20 est monté dans l'enceinte en coopération avec la première ouverture 113, par exemple juste en regard de cette ouverture 113, pour aspirer le gaz porteur à travers cette première ouverture 113 et le refouler par la deuxième ouverture 115.

Quant au barboteur 30, il est constitué par la partie basse du flacon 140 apte à contenir une quantité de liquide volatil 31 déterminée de façon que le niveau 145 de ce liquide volatil soit supérieur à celui de la quatrième ouverture 122 de la tubulure 120 quand le flacon 140 est dans sa seconde position, c'est-à-dire au-dessus du plan horizontal qui contient cette ouverture 122.

Le dispositif de fractionnement 40 disposé dans l'espace annulaire 144 défini ci-avant est avantageusement constitué d'au moins un disque annulaire 146 comprenant une pluralité de percées 147 et un orifice central 149 d'une section complémentaire de la section de la paroi latérale extérieure 123 de la tubulure 120 de façon que cette tubulure 120 traverse le disque annulaire 146 par cet orifice central 149.

Ce disque 146 est représenté sur la figure 2 avec son bord externe 148 solidaire de la paroi latérale intérieure 143 du flacon 140. Mais il sera avantageusement monté solidaire soit de l'enceinte 111 soit de la tubulure 120 (figures 3 et 4), de façon amovible pour pouvoir être facilement nettoyé et pour ne pas gêner le remplissage du flacon 140 comme il sera explicité ci-après. Dans ce cas, il existera un léger espace entre son bord externe 148 et la paroi latérale intérieure 143 du flacon.

Quant à l'organe de traitement 50, il est disposé dans le canal de sortie 160, figures 2 et 3, ce canal de sortie 160 et l'organe de traitement 50 n'apparaissant pas sur la figure 4 puisqu'étant situés derrière, notamment, l'aspirateur 20 et la source d'énergie électrique 100 mentionnée ci-après.

De façon avantageuse, pour obtenir un appareil le plus compact possible, le canal de sortie 160 est défini dans l'enceinte 111, figures 2 et 3, de façon que la sixième ouverture 161 soit réalisée dans le fond 114 de l'enceinte 111, ce canal de sortie 160 traversant la paroi latérale 116 de l'enceinte de façon que la septième ouverture 162 soit située à l'extérieur de cette enceinte 111 dans le milieu ambiant 200 pour y déboucher.

De façon préférentielle, de canal de sortie 160 est situé à l'intérieur de l'enceinte de façon que sa paroi latérale baigne dans le flux du gaz porteur mis en circulation par l'aspirateur 20.

Selon une réalisation possible, l'organe de traitement 50 du flux gazeux chargé fractionné pour lui donner une qualité déterminée, avant qu'il ne soit éjecté à la sortie du conduit fluidique 10, est constitué par au moins l'un des éléments suivants : un générateur d'arc électrique 70 à au moins deux électrodes apte à produire des effluves électriques, un générateur de chaleur 80, un générateur de rayonnement ultra-violet.

Dans le cas d'une réalisation avec un générateur d'arc électrique 70 à au moins deux électrodes apte à produire des effluves électriques, ce dernier comporte, figure 3, un étranglement fluidique 71 constitué d'un manchon 72 à passage central 73 en un matériau électriquement isolant et positionné dans l'axe du canal de sortie 160 de façon étanche sur la paroi latérale intérieure du canal de sortie 160. Le générateur d'arc électrique 70 comporte aussi une âme centrale 74 en un matériau électriquement conducteur chemisant le passage central du manchon, cette âme centrale 74 constituant l'une des deux électrodes, et une borne électrique centrale 75 positionnée dans le canal de sortie 160 en amont du passage central 73 de façon qu'elle soit isolée électriquement de ce canal de sortie 160 et positionnée à une distance déterminée non nulle de l'âme centrale 74, cette borne électrique centrale 75 constituant l'autre électrode, l'arc électrique étant apte à se produire, de façon connue, entre ces deux électrodes 74 et 75.

Dans le cas d'une réalisation avec un générateur de chaleur 80, ce dernier est avantageusement constitué d'une résistance électrique 81 placée dans le canal de sortie 160, figure 3. Cependant, ce générateur de chaleur peut être couplé avec le générateur d'art électrique 70, comme illustré sur la figure 3. Dans ce cas, la résistance électrique 81 est positionnée en aval de du passage central 73.

De façon avantageuse, l'appareil comporte en outre des moyens de guidage 90 du déplacement du flacon 140 quand il passe de sa première position à la seconde et réciproquement sous l'action par exemple des moyens 150 de commande de son déplacement qui ont été définis précédemment.

Selon une réalisation préférentielle, ces moyens de guidage 90 du déplacement du flacon 140 sont constitués, figures 2 et 4, par au moins deux pattes 91, 92 solidaires de l'un des deux éléments "flacon" 140 et "enceinte" 111, et deux glissières 93, 94 dans lesquelles sont aptes à glisser les deux pattes 91, 92 et, réciproquement, respectivement solidaires de l'un des deux éléments : "enceinte" 111 et "flacon" 140.

Selon la réalisation illustré sur la figure 2, les deux pattes 91, 92 sont solidaires du flacon 140 et les deux glissières 93, 94 sont solidaires de l'enceinte 111 qui elle-même est solidaire par exemple d'une embase Emb.

Dans le mode de réalisation industriel illustré selon la figure 4, les pattes 91, 92 sont solidaires de l'embase Emb et les deux glissières 93, 94 sont solidaires du flacon par l'intermédiaire d'un plateau réceptacle 170 dans lequel le flacon 140 est contenu. Cette dernière réalisation permet, par exemple d'obtenir le déplacement du flacon entre ses deux première et seconde positions, mais aussi de le sortir du plateau réceptacle 170 pour notamment le nettoyer après chaque usage et/ou le remplir en toute sécurité avec du liquide volatil 31 comme décrit ci-avant.

Ce liquide volatil 31 est avantageusement un dérivé terpénique d'oléorésines.

De façon préférentielle, le gaz porteur apte à être aspiré par la première ouverture 113 est l'air présent dans le milieu ambiant 200, mais pourrait être un tout autre gaz dont une source serait connectée à cette première ouverture 113.

L'appareil comporte de façon avantageuse une source d'énergie électrique 100 pour alimenter au moins l'un des éléments suivants l'aspirateur 20, les moyens de commande 150 du déplacement du flacon 140, l'organe de traitement 50 du flux gazeux chargé fractionné. Cette source d'énergie électrique est constituée par exemple d'un transformateur ou analogue qui peut être relié au secteur ou à une source autonome comme une batterie ou analogue.

De façon très avantageuse, cette source d'énergie électrique 100 est positionnée dans la tubulure 120, ce qui permet des échanges thermiques entre la source d'énergie et le gaz porteur : ce dernier refroidit la source, et la source commence, elle, à réchauffer le gaz porteur avant qu'il ne traverse le barboteur pour se charger en composé volatil.

Cet appareil est principalement utilisé pour réaliser des réactions de peroxydation intermédiaires permettant la production de catalyseurs, dans différents buts par exemple pour réaliser des inhalations. Aussi, est-il avantageux qu'il comporte en outre une canule articulée 60, figure 3, dont une extrémité 61 est connectée à la sortie 12 du conduit fluidique 10, et un embout respiratoire 63 connecté à l'autre extrémité 62 de la canule 60.

Un tel appareil fonctionne et s'utilise de la façon suivante plus particulièrement dans le but de réaliser une inhalation :

Tout d'abord, le flacon 140 est amené dans sa première position et rempli de la quantité déterminée définie précédemment de l'un des liquides volatils 31 mentionnés auparavant.

Puis le flacon 140 est amené dans sa seconde position et l'aspirateur 20 est mis en marche. Il se produit alors une circulation de gaz porteur, en général de l'air ambiant, qui pénètre dans le circuit fluidique 10 par l'ouverture 113 et suit le trajet défini sur les figures 3 et 4 par des flèches épaisses. Le gaz porteur passe dans le barboteur 30 et se charge en vapeurs du liquide volatil 31, et le flux gazeux chargé est fractionné en passant par le ou les disques percés 146 pour constituer un flux chargé dans une répartition la plus homogène possible.

Le flux gazeux chargé fractionné passe ensuite dans le canal de sortie 160 où il est traité comme, par exemple, décrit dans le document antérieur cité au préambule de la présente description. Il ne comporte plus ou peu par exemple de vapeurs de dérivés terpéniques d'oléorésines non transformées, ni de dérivés ozoniques indésirables.

Ce flux gazeux chargé fractionné et purifié est alors éjecté en sortie 12 du circuit fluidique 10 dans le milieu ambiant 200. Il peut aussi être aspiré via la canule 60 et l'embout respiratoire 63 par une personne qui doit faire une inhalation.

## Revendications

1. Appareil de production, d'un gaz chargé d'un composé volatil, comportant :
• un conduit fluidique (10) comprenant une entrée (11) et une sortie (12), et, montés successivement en série dans ledit conduit fluidique (10) entre son entrée (11) et sa sortie (12) :
• un aspirateur (20) pour aspirer un gaz porteur disponible à l'entrée (11) du conduit fluidique (10) et le refouler vers la sortie (12) du dit conduit fluidique (10),
• un barboteur (30) apte à faire passer le gaz porteur dans un liquide volatil (31) apte à être contenu dans ledit barboteur de façon à obtenir en sortie (32) de ce barboteur un flux du gaz porteur (33) chargé en vapeurs du dit liquide volatil,
• un dispositif de fractionnement (40) du dit flux gazeux chargé obtenu en sortie (32) du dit barboteur (30) pour délivrer à sa sortie (41) ledit flux gazeux chargé sous une forme fractionnée, et
• un organe de traitement (50) du dit flux gazeux chargé fractionné pour lui donner une qualité déterminée, avant qu'il ne soit éjecté à la sortie (12) du conduit fluidique (10), appareil de production **caractérisé par le fait que** ledit conduit fluidique (10) se compose d'au moins :
• d'une enceinte (111) apte à être positionnée verticalement comprenant une première ouverture (113) située sensiblement en sa partie supérieure (112), ladite première ouverture constituant l'entrée (11) du conduit fluidique (10) et un fond (114) relativement plan dans lequel est réalisée au moins une deuxième ouverture (115),
• d'une tubulure (120) d'une hauteur selon la verticale égale à "L", comprenant à ses deux extrémités, respectivement une troisième ouverture (121) située en position haute et une quatrième ouverture (122) située en position basse, ladite tubulure (120) étant solidarisée au dit fond (114) de ladite enceinte (111) de façon que les deuxième et troisième ouvertures (115, 121) soient en communication de façon étanche,
• d'un flacon (140) comportant en partie haute une cinquième ouverture (141) d'une forme sensiblement complémentaire du fond (114) de ladite enceinte (111), ledit flacon ayant une profondeur selon la verticale supérieure à la hauteur "L" de la tubulure (120), et
• d'un canal de sortie (160) comportant une sixième (161) et une septième (162) ouverture, ledit canal de sortie étant monté en coopération avec ladite enceinte (111) et ledit flacon (140) de façon que la sixième ouverture (161) soit apte à être en communication étanche avec la cinquième ouverture (141) et que la septième ouverture (162) débouche dans le milieu ambiant (200), cette septième ouverture (160) constituant la sortie (12) du dit conduit fluidique (10).

2. Appareil selon la revendication 1, **caractérisé par le fait qu'**il comporte en outre des moyens (150) pour commander le déplacement du dit flacon (140) entre deux première et seconde positions stables, la première position étant une position dans laquelle la cinquième ouverture (141) du flacon est éloignée du fond (114) de ladite enceinte (111) et la seconde position étant celle dans laquelle ladite cinquième ouverture (141) du flacon (140) est sensiblement confondue de façon étanche avec le fond (114) de ladite enceinte (111), de façon que la tubulure (120) plonge dans ledit flacon (140) sans atteindre son fond (142) pour laisser, entre sa quatrième ouverture (122) et le fond (142) du flacon, une distance non nulle, et de façon en outre que, entre la paroi latérale extérieure (123) de la tubulure (120) et la paroi latérale intérieure (143) du flacon (140), soit défini un espace annulaire (144).

3. Appareil selon la revendication 2, **caractérisé par le fait que** :
• ledit aspirateur (20) est monté dans ladite enceinte en coopération avec ladite première ouverture (113) pour aspirer à travers cette première ouverture (113) le gaz porteur et le refouler par la deuxième ouverture (115),
• ledit barboteur (30) est constitué par la partie basse du flacon (140) apte à contenir une quantité de liquide volatil (31) déterminée de façon que le niveau (145) du dit liquide volatil soit supérieur à celui de la quatrième ouverture (122) de la tubulure (120) quand le flacon (140) est dans sa seconde position,
• ledit dispositif de fractionnement (40), disposé dans l'espace annulaire (144), est constitué par au moins un disque annulaire (146) comprenant une pluralité de percées (147) et un orifice central (149) d'une section complémentaire de la section de la paroi latérale extérieure (123) de la tubulure (120), la tubulure (120) passant dans ledit orifice central, et que
• ledit organe de traitement (50) est disposé dans ledit canal de sortie (160).

4. Appareil selon l'une des revendications 1 à 3, **caractérisé par le fait que** le canal de sortie (160) est défini dans ladite enceinte (111) de façon que la sixième ouverture (161) soit réalisée dans le fond (114) de ladite enceinte (111), ce canal de sortie (160) traversant la paroi latérale (116) de l'enceinte de façon que la septième ouverture (162) soit située à l'extérieur de ladite enceinte (111) dans le milieu ambiant (200).

5. Appareil selon l'une des revendications 1 à 4, **caractérisé par le fait que** ledit organe de traitement (50) du dit flux gazeux chargé fractionné pour lui donner une qualité déterminée, avant qu'il ne soit éjecté à la sortie du conduit fluidique (10), est constitué par au moins l'un des éléments suivants: un générateur d'arc électrique (70) à au moins deux électrodes, un générateur de chaleur (80), un générateur de rayonnement ultra-violet.

6. Appareil selon la revendication 5, **caractérisé par le fait que** ledit générateur d'arc électrique (70) à au moins deux électrodes comporte un étranglement fluidique (71) constitué d'un manchon (72) à passage central (73) en un matériau électriquement isolant et positionné de façon coaxiale et étanche sur la paroi latérale intérieure du canal de sortie (160) et de façon que ledit flux chargé fractionné passe par le passage central (73) de ce manchon (72), une âme centrale (74) en un matériau électriquement conducteur chemisant le passage central du manchon, cette âme centrale constituant l'une des deux électrodes, et une borne électrique centrale (75) positionnée dans ledit canal de sortie (160) en amont du dit passage central (73) de façon qu'elle soit isolée électriquement de ce canal de sortie (160) et positionnée à une distance déterminée non nulle de l'âme centrale (74), cette borne électrique centrale (75) constituant l'autre électrode.

7. Appareil selon l'une des revendications 5 et 6, **caractérisé par le fait que** ledit générateur de chaleur (80) est constitué d'une résistance électrique (81).

8. Appareil selon l'une des revendications précédentes quand elle dépend de la revendication 2, **caractérisé par le fait qu'**il comporte des moyens de guidage (90) du déplacement du dit flacon (140) entre ses deux première et seconde positions stables.

9. Appareil selon la revendication 8, **caractérisé par le fait que** les moyens de guidage (90) du déplacement du dit flacon sont constitués par au moins deux pattes (91, 92) solidaires de l'un des deux éléments "flacon" (140) et "enceinte" (111), et deux glissières (93, 94) dans lesquelles sont aptes à glisser les deux pattes (91, 92) et, réciproquement, respectivement solidaires de l'un des deux éléments : "enceinte" (111) et "flacon" (140).

10. Appareil selon les revendications précédentes, **caractérisé par le fait qu'**il comporte une source d'énergie électrique (100) pour alimenter au moins l'un des éléments suivants : l'aspirateur (20), les moyens de commande (150) du déplacement du flacon (140), l'organe de traitement (50), cette source d'énergie électrique (100) étant positionnée dans la tubulure (120).

11. Appareil selon l'une des revendications précédentes, **caractérisé par le fait qu'**il comporte en outre une canule articulée (60) dont une extrémité (61) est connectée à la sortie (12) du conduit fluidique (10), et un embout respiratoire (63) connecté à l'autre extrémité (62) de ladite canule.

12. Appareil selon l'une des revendications précédentes, **caractérisé par le fait que** ledit liquide volatil est une essence de dérivés terpéniques d'oléorésines et que le gaz porteur est l'air ambiant.

## Patentansprüche

1. Vorrichtung für die Produktion eines mit einer flüchtigen Verbindung angereicherten Gases, die umfasst:
- eine Fluidleitung (10), die einen Einlass (11) und einen Auslass (12) aufweist, wobei nacheinander in Reihe in der Fluidleitung (10) zwischen deren Eingang (11) und deren Ausgang (12) montiert sind:
- eine Saugvorrichtung (20), um ein am Eingang (11) der Fluidleitung (10) verfügbares Trägergas anzusaugen und zum Ausgang (12) der Fluidleitung (10) zu fördern;
- einen Gaswäscher (30), der das Trägergas in eine flüchtige Flüssigkeit (31) bewegen kann, die in dem Gaswäscher enthalten sein kann, derart, dass am Ausgang (32) dieses Gaswäschers ein Trägergasstrom (33), der mit Dämpfen der flüchtigen Flüssigkeit angereichert ist, erhalten wird,
- eine Fraktionierungsvorrichtung (40) für den am Ausgang (32) des Gaswäschers (30) erhaltenen angereicherten Gasstrom, um an ihrem Ausgang (41) den angereicherten Gasstrom in einer fraktionierten Form auszugeben, und
- ein Behandlungsorgan (50) für den fraktionierten, angereicherten Gasstrom, um ihm eine bestimmte Qualität zu verleihen, bevor er am Ausgang (12) der Fluidleitung (10) ausgestoßen wird, wobei die Produktionsvorrichtung **dadurch gekennzeichnet ist, dass** die Fluidleitung (10) wenigstens gebildet ist aus:
- einem Behälter (111), der vertikal positioniert sein kann und eine erste Öffnung (113), die sich im Wesentlichen in seinem oberen Teil (112) befindet, wobei die erste Öffnung den Eingang (11) der Fluidleitung (10) bildet, und einen Boden (114), der verhältnismäßig eben ist und in dem wenigstens eine zweite Öffnung (115) verwirklicht ist, aufweist,
- einer Rohranordnung (120) mit einer Höhe längs der Vertikalen, die gleich "L" ist, die an ihren zwei Enden eine dritte Öffnung (121), die sich in der oberen Position befindet, bzw. eine vierte Öffnung (122), die sich in der unteren Position befindet, aufweist, wobei die Rohranordnung (120) mit dem Boden (114) des Behälters (111) in der Weise fest verbunden ist, dass die zweite und die dritte Öffnung (115, 121) auf dichte Weise kommunizieren,
- einem Fläschchen (140), das in seinem oberen Teil eine fünfte Öffnung (141) mit einer zu dem Boden (114) des Behälters (111) im Wesentlichen komplementären Form aufweist, wobei das Fläschchen längs der Vertikalen eine Tiefe besitzt, die größer als die Höhe "L" der Rohranordnung (120) ist, und
- einem Ausgangskanal (160), der eine sechste (161) und eine siebte (162) Öffnung aufweist, wobei der Ausgangskanal zusammenwirkend mit dem Behälter (111) und dem Fläschchen (140) in der Weise montiert ist, dass die sechste Öffnung (161) mit der fünften Öffnung (141) dicht kommunizieren kann und dass die siebte Öffnung (162) in das umgebende Medium (200) mündet, wobei diese siebte Öffnung (160) den Ausgang (12) der Fluidleitung (10) bildet.

2. Vorrichtung nach Anspruch 1, **dadurch gekennzeichnet, dass** sie außerdem Mittel (150) umfasst, um die Verlagerung des Fläschchens (140) zwischen zwei stabilen Positionen, einer ersten und einer zweiten Position, zu steuern, wobei die erste Position eine Position ist, in der die fünfte Öffnung (141) des Fläschchens vom Boden (114) des Behälters (111) entfernt ist, und wobei die zweite Position jene ist, in der sich die fünfte Öffnung (141) des Fläschchens (140) auf dichte Weise an den Boden (114) des Behälters (111) im Wesentlichen anschmiegt, derart, dass die Rohranordnung (120) in das Fläschchen (140) eintaucht, ohne seinen Boden (142) zu erreichen, um zwischen ihrer vierten Öffnung (122) und dem Boden (142) des Fläschchens einen von null verschiedenen Abstand zu lassen, und derart, dass außerdem zwischen der äußeren Seitenwand (123) der Rohranordnung (120) und der inneren Seitenwand (143) des Fläschchens (140) ein Ringraum (144) definiert ist.

3. Vorrichtung nach Anspruch 2, **dadurch gekennzeichnet, dass**:
- die Saugvorrichtung (20) in dem Behälter zusammenwirkend mit der ersten Öffnung (113) montiert ist, um durch diese erste Öffnung (113) das Trägergas anzusaugen und es durch die zweite Öffnung (115) zu fördern,
- der Gaswäscher (30) durch den unteren Teil des Fläschchens (140), der eine bestimmte Menge einer flüchtigen Flüssigkeit (31) enthalten kann, gebildet ist, derart, dass der Pegel (145) der flüchtigen Flüssigkeit höher ist als die Höhe der vierten Öffnung (122) der Rohranordnung (120), wenn sich das Fläschchen (140) in seiner zweiten Position befindet,
- die Fraktionierungsvorrichtung (40), die in dem Ringraum (144) angeordnet ist, durch wenigstens eine Ringscheibe (146) gebildet ist, die mehrere Durchlochungen (147) und eine mittige Öffnung (149) mit einem zum Querschnitt der äußeren Seitenwand (123) der Rohranordnung (120) komplementären Querschnitt aufweist, und dass
- das Behandlungsorgan (50) in dem Ausgangskanal (160) angeordnet ist.

4. Vorrichtung nach einem der Ansprüche 1 bis 3, **dadurch gekennzeichnet, dass** der Ausgangskanal (160) in dem Behälter (111) in der Weise definiert ist, dass die sechste Öffnung (161) im Boden (114) des Behälters (111) verwirklicht ist, wobei dieser Ausgangskanal (160) die Seitenwand (116) des Behälters in der Weise durchquert, dass sich die siebte Öffnung (162) außerhalb des Behälters (111) in dem umgebenden Milieu (200) befindet.

5. Vorrichtung nach einem der Ansprüche 1 bis 4, **dadurch gekennzeichnet, dass** das Behandlungsorgan (50) für den fraktionierten, angereicherten Gasstrom, um ihm eine bestimmte Qualität zu verleihen, bevor es aus dem Ausgang der Fluidleitung (10) ausgestoßen wird, durch wenigstens eines der folgenden Elemente gebildet ist: einen Lichtbogengenerator (70) mit wenigstens zwei Elektroden, einen Wärmegenerator (80) und einen Ultraviolettstrahlengenerator.

6. Vorrichtung nach Anspruch 5, **dadurch gekennzeichnet, dass** der Lichtbogengenerator (70) mit wenigstens zwei Elektroden eine Fluidverengung (71), die aus einer Muffe (72) mit einem mittleren Durchgang (73) und aus einem elektrisch isolierenden Material gebildet ist und koaxial und dicht an der inneren Seitenwand des Ausgangskanals (160) und in der Weise positioniert ist, dass sich der fraktionierte, angereicherte Strom durch den mittigen Durchlass (73) dieser Muffe (72) bewegt, einen mittigen Kern (74) aus einem elektrisch leitenden Material, das den mittigen Durchlass der Muffe auskleidet, wobei dieser mittige Kern eine der zwei Elektroden bildet, und einen mittigen elektrischen Anschluss (75), der in dem Ausgangskanal (160) stromaufseitig des mittigen Durchlasses (73) in der Weise positioniert ist, dass er von diesem Ausgangskanal (160) elektrisch isoliert ist, und in einem von null verschiedenen Abstand von dem mittigen Kern (74) positioniert ist, wobei dieser mittige elektrische Anschluss (75) die andere Elektrode bildet, umfasst.

7. Vorrichtung nach einem der Ansprüche 5 und 6, **dadurch gekennzeichnet, dass** der Wärmegenerator (80) aus einem elektrischen Widerstand (81) gebildet ist.

8. Vorrichtung nach einem der vorhergehenden Ansprüche, wenn abhängig von Anspruch 2, **dadurch gekennzeichnet, dass** sie Mittel (90) für die Führung der Verlagerung des Fläschchens (140) zwischen seiner ersten und seiner zweiten stabilen Position umfasst.

9. Vorrichtung nach Anspruch 8, **dadurch gekennzeichnet, dass** die Mittel (90) zum Führen der Verlagerung des Fläschchens durch wenigstens zwei Laschen (91, 92), die mit einem der zwei Elemente "Fläschchen" (140) und "Behälter" (111) fest verbunden sind, und aus zwei Gleitschienen (93, 94), in denen die zwei Laschen (91, 92) gleiten können, oder umgekehrt, die mit jeweils einem der zwei Elemente: "Behälter" (111) bzw. "Fläschchen" (140) fest verbunden sind, gebildet sind.

10. Vorrichtung nach den vorhergehenden Ansprüchen, **dadurch gekennzeichnet, dass** sie eine Quelle (100) für elektrische Energie umfasst, um wenigstens eines der folgenden Elemente zu versorgen: Saugvorrichtung (20), Mittel (150) zum Steuern der Verlagerung des Fläschchens (140), Bearbeitungsorgan (50), wobei diese Quelle (100) für elektrische Energie in der Rohranordnung (120) positioniert ist.

11. Vorrichtung nach einem der vorhergehenden Ansprüche, **dadurch gekennzeichnet, dass** sie außerdem ein angelenktes Röhrchen (60) umfasst, wovon ein Ende (61) mit dem Ausgang (12) der Fluidleitung (10) verbunden ist, und einen Atmungsstutzen (63), der mit dem anderen Ende (62) des Röhrchens verbunden ist, umfasst.

12. Vorrichtung nach einem der vorhergehenden Ansprüche, **dadurch gekennzeichnet, dass** die flüchtige Flüssigkeit eine Essenz von Terpenderivaten von Terpentinen ist und dass das Trägergas die Umgebungsluft ist.

## Claims

1. Apparatus for producing a gas charged with a volatile compound, the apparatus comprising:
· a fluid-flow duct (10) having an inlet (11) and an outlet (12), and mounted in succession in series in said fluid-flow duct (10) between its inlet (11) and its outlet (12):
· a suction fan (20) for sucking in a carrier gas available at the inlet (11) of the fluid-flow duct (10) and for discharging it towards the outlet (12) of said fluid-flow duct (10);
· a bubbler (30) suitable for causing the carrier gas to pass through a volatile liquid (31) suitable for being contained in said bubbler so as to obtain at the outlet (32) of the bubbler a stream of carrier gas (33) charged with vapor of said volatile liquid;
· a fractioning device (40) for fractioning said charged gas stream obtained at the outlet (32) of said bubbler (30) so as to deliver at its outlet (41) said charged gas stream in fractioned form; and
· a treatment member (50) for treating said fractioned charged gas stream to impart a determined quality thereto prior to it being ejected from the outlet (12) of the fluid-flow duct (10), apparatus for production **characterized by** the fact that said fluid-flow duct (10) comprises at least:
· an enclosure (111) suitable for being positioned vertically, having both a first opening (113) situated substantially in its top portion (112), said first opening constituting the inlet (11) of the fluid-flow duct (10), and a relatively plane bottom (114) having at least one second opening (115) formed therein;
· a tube (120) of vertical height equal to "L", having at its two ends respectively a third opening (121) situated in a high position and a fourth opening (122) situated in a low position, said tube (120) being secured to said bottom (114) of said enclosure (111) in such a manner that the second and third openings (115, 121) are in leaktight communication;
· a flask (140) having in its upper portion a fifth opening (141) of a shape that is substantially complementary to the bottom (114) of said enclosure (111), said flask having a depth in the vertical direction that is greater than the height "L" of the tube (120); and
· an outlet channel (160) having sixth and seventh openings (161, 162), said outlet channel being mounted to co-operate with said enclosure (111) and said flask (140) in such a manner that the sixth opening (161) is suitable for being in leaktight communication with the fifth opening (141), and that the seventh opening (162) opens out into the ambient medium (200), said seventh opening (160) constituting the outlet (12) of said fluid-flow duct (10).

2. Apparatus according to claim 1, **characterized by** the fact that it further includes means (150) for controlling the movement of said flask (140) between first and second stable positions, the first position being a position in which the fifth opening (141) of the flask is spaced apart from the bottom (114) of said enclosure (111), and the second position being a position in which said fifth opening (141) of the flask (140) substantially coincides in leaktight manner with the bottom (114) of said enclosure (111), in such a manner that the tube (120) dips into said flask (140) without reaching its bottom (142) so as to leave a non-zero distance between its fourth opening (122) and the bottom (142) of the flask, and also in such a manner as to define an annular space (144) between the outer side wall (123) of the tube (120) and the inner side wall (143) of the flask (140).

3. Apparatus according to claim 2, **characterized by** the facts that:
· said suction fan (20) is mounted in said enclosure to co-operate with said first opening (113) to suck the carrier gas in through said first opening (113) and to discharge it through the second opening (115);
· said bubbler (30) is constituted by the bottom portion of the flask (140) that is suitable for containing a determined quantity of volatile liquid (31) so that the level (145) of said volatile liquid is higher than the level of the fourth opening (122) of the tube (120) when the flask (140) is in its second position;
· said fractioning device (40) located in the annular space (144) is constituted by at least one annular disk (146) including a plurality of holes (147), and a central orifice (149) of section complementary to the section of the outer side wall (123) of the tube (120), the tube (120) passing through said central orifice; and
· said treatment member (50) is disposed in said outlet channel (160).

4. Apparatus according to any one of claims 1 to 3, **characterized by** the fact that the outlet channel (160) is defined in said enclosure (111) in such a manner that the sixth opening (161) is formed in the bottom (114) of said enclosure (111), the outlet channel (160) passing through the side wall (116) of the enclosure in such a manner that the seventh opening (162) is situated outside said enclosure (111) in the ambient medium (200).

5. Apparatus according to any one of claims 1 to 4, **characterized by** the fact that said treatment member (50) for treating said fractioned charged gas stream so as to impart a determined quality thereto prior to it being ejected through the outlet of the fluid-flow duct (10) is constituted by at least one of the following elements: an electric arc generator (70) having at least two electrodes; a heat generator (80); and an ultraviolet radiation generator.

6. Apparatus according to claim 5, **characterized by** the fact that said electric arc generator (70) having at least two electrodes includes both a fluid-flow constriction (71) constituted by a sleeve (72) that has a central passage (73), that is made of an electrically-insulating material, and that is positioned coaxially and in leaktight manner against the inner side wall of the outlet channel (160) in such a manner that said fractioned charged stream passes through the central passage (73) of said sleeve (72), a central core (74) of an electrically-conductive material lining the central passage of the sleeve, said central core constituting one of the two electrodes, and a central electrical terminal (75) positioned in said outlet channel (160) upstream from said central passage (73) in such a manner as to be electrically isolated from said outlet channel (160) and to be positioned at a determined non-zero distance from the central core (74), said central electrical terminal (75) constituting the other electrode.

7. Apparatus according to claim 5 or claim 6, **characterized by** the fact that said heat generator (80) is constituted by an electrical resistance (81).

8. Apparatus according to any preceding claim when dependent on claim 2, **characterized by** the fact that it includes guide means (90) for guiding the movement of said flask (140) between its first and second stable positions.

9. Apparatus according to claim 8, **characterized by** the fact that the guide means (90) for guiding the movement of said flask are constituted by at least two tabs (91, 92) that are secured to one of the two elements constituted by the flask (140) and the enclosure (111), and by two slideways (93, 94) in which the two tabs (91, 92) are suitable for sliding and that are secured respectively to the other one of the two elements constituted by the enclosure (111) and the flask (140).

10. Apparatus according to any preceding claim, **characterized by** the fact that it includes an electrical power supply (100) for powering at least one of the following elements: the suction fan (20); the control means (150) for controlling the movement of the flask (140); and the treatment member (50); the electrical power supply (100) being positioned in the tube (120).

11. Apparatus according to any preceding claim, **characterized by** the fact that it further includes a hinged pipe (60) having one end (61) connected to the outlet (12) of the fluid-flow duct (10), and having a breathing endpiece (63) connected to the other end (62) of said pipe.

12. Apparatus according to any preceding claim, **characterized by** the fact that said volatile liquid is an essence of oleoresin terpene derivatives, and the carrier gas is ambient air.
